(19) Europäisches Patentamt
European Patent Office
Office européen des brevets

(11) **EP 2 950 746 B1**

(12) **FASCICULE DE BREVET EUROPEEN**

(45) Date de publication et mention
de la délivrance du brevet:
**17.03.2021 Bulletin 2021/11**

(21) Numéro de dépôt: **14702261.0**

(22) Date de dépôt: **31.01.2014**

(51) Int Cl.:
***A61F 2/00*** *(2006.01)*

(86) Numéro de dépôt international:
**PCT/EP2014/051943**

(87) Numéro de publication internationale:
**WO 2014/118335 (07.08.2014 Gazette 2014/32)**

(54) **SYSTEME DE DETECTION D'UN DISPOSITIF ENDO-URETRAL POUR UN SPHINCTER ARTIFICIEL IMPLANTABLE DANS LE CORPS HUMAIN OU ANIMAL**

**SYSTEM ZUR ERKENNUNG EINER ENDOURETHRALEN VORRICHTUNG FÜR EINEN IN DEN KÖRPER EINES MENSCHEN ODER EINE TIERES IMPLANTIERBAREN KÜNSTLICHEN SCHLIESSMUSKEL**

**SYSTEM FOR DETECTING AN ENDOURETHRAL DEVICE FOR AN ARTIFICIAL SPHINCTER THAT IS IMPLANTABLE IN THE BODY OF A HUMAN OR ANIMAL**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.02.2013 FR 1350880**

(43) Date de publication de la demande:
**09.12.2015 Bulletin 2015/50**

(73) Titulaires:
• **Uromems**
**38000 Grenoble (FR)**
• **Assistance Publique Hôpitaux De Paris**
**75004 Paris (FR)**

(72) Inventeurs:
• **LAMRAOUI, Hamid**
**F-38000 Grenoble (FR)**
• **MOZER, Pierre**
**F-94300 Vincennes (FR)**

(74) Mandataire: **Regimbeau**
**87 rue de Sèze**
**69477 Lyon Cedex 06 (FR)**

(56) Documents cités:
**WO-A1-2009/027196     WO-A2-01/50833**
**WO-A2-03/043534     US-A1- 2008 300 449**
**US-A1- 2012 157 759**

EP 2 950 746 B1

## Description

## DOMAINE DE L'INVENTION

L'invention concerne un système de détection d'un dispositif endo-urétral pour un sphincter artificiel implantable dans le corps humain ou animal.

## ARRIERE PLAN DE L'INVENTION

Le traitement de l'incontinence urinaire peut impliquer l'implantation, chez un patient, d'un sphincter artificiel.

Un tel sphincter comprend typiquement une manchette occlusive placée autour de l'urètre (chez l'homme ou la femme) ou parfois du col vésical (chez la femme) ou de la prostate chez l'homme dans le but d'exercer une compression directe ou indirecte sur l'urètre afin d'éviter des fuites urinaires, un dispositif d'actionnement de ladite manchette pour faire varier la compression exercée sur l'urètre ou le col vésical, ainsi qu'une unité de commande du dispositif d'actionnement.

Un tel sphincter artificiel est décrit en particulier dans [1].

Différentes technologies de sphincters artificiels ont été proposées, basées notamment sur différents types de manchettes occlusives et du mode d'actionnement associé.

Selon une forme d'exécution, la manchette occlusive est une manchette gonflable remplie d'un fluide.

Le dispositif de compression est un dispositif hydraulique, comprenant un réservoir dudit fluide et un actionneur électromécanique permettant d'ajouter ou de retirer ledit fluide pour compresser ou décompresser la partie qui occlut.

Un tel sphincter artificiel est décrit par exemple dans [2].

Un autre exemple de sphincter artificiel est décrit dans [3].

Il existe également un sphincter basé sur un fonctionnement mécanique, dans lequel la manchette occlusive est une bande entourant l'urètre ou le col vésical et reliée à une sangle qui permet d'exercer une tension mécanique plus ou moins forte sur la bande à l'aide d'un actionneur électromécanique [4].

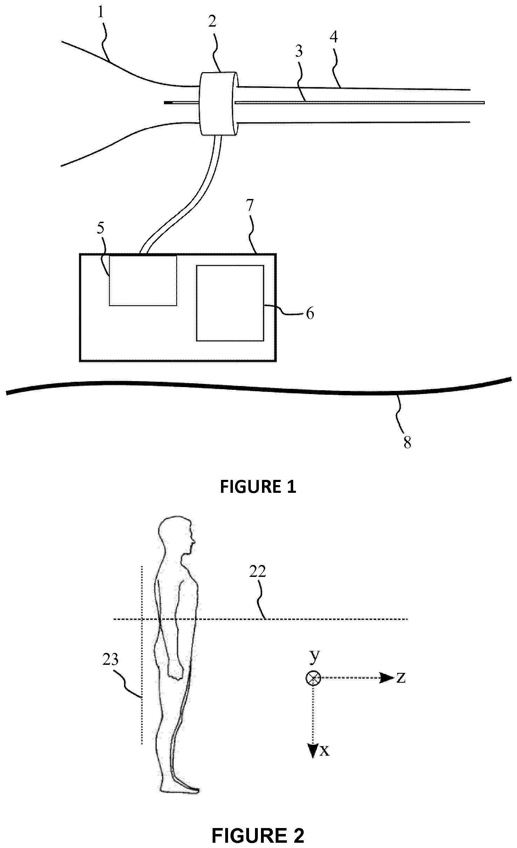

Lorsque le patient doit subir un geste endo-urétral, typiquement un sondage urétral, dans lequel, comme illustré à la figure 1, on introduit dans l'urètre une sonde jusqu'à la vessie, une pression très forte est exercée sur l'urètre dans la région entourée par la manchette occlusive.

Il est donc recommandé aux praticiens de relâcher la pression exercée par la manchette avant de procéder à l'insertion de l'instrument ou de la sonde et pendant tout le temps de son insertion en désactivant le sphincter artificiel [5].

En effet, en l'absence d'une telle précaution, cette pression excessive est responsable du phénomène d'érosion de l'urètre en créant une brèche dans sa paroi conduisant ainsi la manchette à se retrouver partiellement ou totalement dans la lumière de l'urètre.

Cette érosion de l'urètre implique une intervention chirurgicale pour retirer la manchette voire de l'ensemble du sphincter artificiel. Une fois la cicatrisation de l'urètre obtenue, ce qui nécessite plusieurs mois, un nouveau sphincter artificiel peut alors être implanté.

L'érosion urétrale suite à un geste endo-urétral, et tout particulièrement à la mise en place d'une sonde est une cause importante de révision de l'implant.

Parmi les cas de révision liés à une érosion urétrale, plus de la moitié est due à un sondage urétral effectué alors que la manchette comprimait l'urètre [6].

A cet égard, certains cliniciens estiment que la sonde endommage l'urètre dès son passage dans la région entourée par la manchette occlusive ; d'autres cliniciens pensent en revanche que l'érosion se produit lorsque la compression excessive est appliquée au-delà d'une certaine durée.

En outre, l'insertion de la sonde peut être la cause d'une détérioration de la manchette occlusive et/ou du dispositif d'actionnement associé due à la pression très forte engendrée.

En tout état de cause, il est souhaitable, lors d'un geste endo-urétral tel qu'un sondage ou l'introduction d'un fibroscope par exemple, que la compression de l'urètre soit relâchée dès l'insertion du dispositif endo-urétral et pendant toute la durée de présence de celui-ci dans l'urètre.

En principe, le patient ou le personnel soignant est en mesure de commander lui-même la décompression de la manchette par des moyens de commande prévus dans le sphincter artificiel.

Cependant, il existe des situations dans lesquelles cette décompression préalable n'est pas possible.

Tel est le cas par exemple dans le cas d'une intervention chirurgicale pratiquée en urgence sur un patient, le personnel soignant n'ayant pas nécessairement connaissance de la présence du sphincter artificiel implanté.

Pour garantir que, même dans une telle situation, le geste endo-urétral n'endommage pas l'urètre, on cherche donc à provoquer la décompression automatique de la manchette dès l'insertion du dispositif.

Cependant, afin d'éviter des fuites urinaires intempestives, il est nécessaire que cette décompression ne soit provoquée que lorsqu'un dispositif endo-urétral est réellement introduit dans l'urètre.

Or, il existe des situations dans lesquelles la pression exercée sur l'urètre est très élevée sans qu'une sonde urétrale n'ait été introduite.

Ainsi, lorsque le patient est assis, il est possible qu'il appuie fortement sur son périnée, ce qui engendre une augmentation de la compression du même ordre que celle provoquée par l'introduction d'une sonde.

Une simple mesure de la compression de l'urètre ne permet donc pas de détecter de manière fiable un geste endo-urétral. Un système de détection d'un dispositif endo-urétral pour un sphincter urinaire artificiel selon le préambule de la revendication 1 est connu du document WO-A-01/50833.

**BREVE DESCRIPTION DE L'INVENTION**

Un but de l'invention est donc de proposer un système de détection pour un sphincter artificiel implantable qui permette d'ordonner la décompression immédiate de la manchette occlusive dès que l'introduction d'un dispositif à l'intérieur de l'urètre est détectée tout en minimisant le risque de détection erronée.

Un autre but de l'invention est de permettre d'adapter la compression de la manchette en fonction du patient, pour éviter des fuites entre le dispositif endourétral et la paroi de l'urètre dans le cas de patients en particulier paraplégiques qui sont porteurs de sphincter artificiel et qui doivent régulièrement se sonder.

De préférence, l'invention doit en outre permettre d'éviter tout risque d'endommagement du dispositif d'actionnement de la manchette sous l'effet des contraintes importantes exercées sur celui-ci par le dispositif endo-urétral.

Conformément à l'invention, il est proposé un système de détection pour un sphincter urinaire artificiel implantable dans le corps d'un patient, ledit sphincter comprenant une manchette occlusive adaptée pour comprimer l'urètre, respectivement le col vésical ou la prostate dudit patient, un dispositif d'actionnement de ladite manchette pour ajuster la compression exercé par la manchette et une unité de commande adaptée pour commander le dispositif d'actionnement.

Ledit système de détection comprend :

- au moins un capteur dit « capteur de compression » adapté pour mesurer un paramètre de compression de l'urètre, respectivement du col vésical ou de la prostate,
- au moins un capteur dit « capteur de posture » adapté pour mesurer un paramètre de posture du patient, et
- une unité de traitement configurée pour :

  - recevoir des données de mesure desdits capteurs,
  - déterminer, à partir des données de mesure du paramètre de compression de l'urètre, respectivement du col vésical :

    (i) si le paramètre de compression de l'urètre, respectivement du col vésical, dépasse un seuil prédéterminé pendant une durée prédéterminée, sans qu'un ordre de ladite unité de commande soit envoyé au dispositif d'actionnement pour réduire la compression exercée par la manchette,

  - déterminer, à partir des données de mesure du paramètre de posture du patient :
    (ii) si le patient est dans une position allongée,
  - si les conditions (i) et (ii) sont remplies, émettre, à destination du dispositif d'actionnement du sphincter urinaire artificiel, un ordre de réduction immédiate de la compression exercée par la manchette occlusive sur l'urètre, respectivement sur le col vésical ou la prostate, un organe de réduction des contraintes mécaniques subies par le dispositif d'actionnement conçu pour, lorsque les contraintes mécaniques subies par le dispositif d'actionnement dépassent un seuil de contraintes déterminé, absorber une partie desdites contraintes de sorte à réduire les contraintes subies par le dispositif d'actionnement tout en maintenant la compression exercé par la manchette au dessus du seuil de détection.

Par « paramètre de compression de l'urètre », on entend toute grandeur mesurable représentative de la compression exercée par la manchette sur l'urètre. Cette grandeur peut résulter d'une mesure directe sur l'urètre mais peut également résulter d'une mesure indirecte. Par exemple, lorsque la manchette occlusive est une manchette hydraulique, la mesure de la pression dans la manchette - voire en un autre point du système hydraulique d'actionnement de la manchette - permet d'évaluer la compression de l'urètre.

Par « paramètre de posture » on entend toute grandeur mesurable représentative de la posture du patient, notamment du fait qu'il soit ou non en position allongée. En effet, lorsque le patient subit un sondage urétral il est placé en position allongée. Cette grandeur peut par exemple résulter d'une mesure accélérométrique indiquant l'orientation du corps du patient par rapport à la gravité terrestre.

Par « réduction de la compression exercé par la manchette », on entend soit une diminution de la compression exercée jusqu'à une valeur non nulle, soit une libération totale de la manchette, correspondant à une compression nulle.

De manière particulièrement avantageuse, ledit au moins un capteur de posture et l'unité de traitement sont configurés pour déterminer si le patient est dans la position allongée (plus particulièrement en décubitus dorsal, ou bien dans certains cas en position décubitus déclive ou en position gynécologique) et pour calculer l'accélération dudit patient afin de déterminer si ledit patient est sensiblement immobile.

De préférence, ledit au moins un capteur de posture est choisi parmi un accéléromètre, un gyroscope et un inclinomètre.

De manière avantageuse, le capteur de compression est adapté pour mesurer un paramètre de fonctionnement du dispositif d'actionnement de la manchette et l'unité de traitement est configurée pour déterminer, à partir de la mesure dudit paramètre, la compression de l'urètre.

Selon un mode de réalisation, le dispositif d'actionnement de la manchette est un dispositif hydraulique adapté pour ajuster une pression de fluide dans la manchette occlusive.

Dans ce cas, ledit au moins un capteur de compression est avantageusement un capteur de pression agencé pour mesurer la pression de fluide dans ledit dispositif hydraulique.

Selon un autre mode de réalisation, le dispositif d'actionnement de la manchette est un dispositif adapté pour ajuster une tension mécanique de la manchette occlusive.

Dans ce cas, le capteur de compression est de préférence un capteur de contrainte mécanique.

Selon un mode de réalisation particulièrement avantageux, le système de détection comprend en outre un organe de réduction des contraintes mécaniques subies par le dispositif d'actionnement.

Selon une forme d'exécution particulière de l'invention, dans le cas d'un dispositif d'actionnement hydraulique, ledit organe comprend une chambre d'expansion agencée dans le dispositif hydraulique de sorte à transférer une partie du volume de fluide dans ladite chambre d'expansion de manière à réduire à une valeur définie la pression dans le dispositif hydraulique.

Selon une forme d'exécution avantageuse, l'unité de traitement est intégrée à l'unité de commande du sphincter urinaire artificiel.

De préférence, l'unité de traitement ou l'unité de commande est configurée pour émettre un signal sonore avant la réduction de la compression exercée par la manchette.

Par ailleurs, l'unité de traitement comprend avantageusement une mémoire sur laquelle est enregistré un programme d'ordinateur, ledit programme comprenant des instructions pour la mise en oeuvre des étapes suivantes :

(S101) la lecture d'un signal d'un paramètre de compression de l'urètre, respectivement du col vésical,
(S102) la comparaison dudit signal avec un seuil prédéterminé,
(S105) si le paramètre de compression de l'urètre, respectivement du col vésical ou de la prostate, dépasse ledit seuil prédéterminé pendant une durée prédéterminée, la lecture d'un signal d'un paramètre de posture du patient, sinon retour à l'étape S101,
(S107) la comparaison d'un paramètre dudit signal avec un plafond prédéterminé, et la détermination d'une position immobile du patient si ledit paramètre est inférieur audit plafond,
(S109) la détermination, à partir dudit signal, de la position du patient en décubitus dorsal,
(S110) la répétition des étapes précédentes pendant un nombre prédéterminé d'itérations,
(S114) l'émission, à destination du dispositif d'actionnement du sphincter urinaire artificiel, d'un ordre de réduction immédiate de la compression exercée par la manchette occlusive sur l'urètre, respectivement sur le col vésical.

De manière optionnelle, ledit au moins un capteur de posture et l'unité de traitement sont configurés pour déterminer si le patient est dans une position assise et pour déterminer si le buste dudit patient est sensiblement immobile.

Dans ce cas, la condition (ii) pour l'émission de l'ordre de réduction immédiate de la compression exercée par la manchette est lorsque le patient est assis.

Ceci permet de tenir compte utilement du cas particulier des patients paraplégiques.

Enfin, de manière avantageuse, l'unité de traitement est configurée pour, si ledit paramètre de compression de l'urètre dépasse un seuil de sécurité prédéterminé supérieur au seuil par rapport auquel ledit paramètre est comparé dans l'évaluation de la condition (i), émettre un ordre de réduction immédiate de la compression exercée par la manchette, quelle que soit le statut de la condition (ii).

Un autre objet concerne un sphincter urinaire artificiel comprenant un système de détection tel que décrit ci-dessus.

Ledit sphincter comprend :

- une manchette occlusive adaptée pour comprimer l'urètre, respectivement le col vésical ou la prostate d'un patient dans lequel il est destiné à être implanté,
- un dispositif d'actionnement de ladite manchette pour ajuster la compression exercée par la manchette,
- une unité de commande adaptée pour commander le dispositif d'actionnement, et
- un système de détection tel que décrit ci-dessus, qui communique avec le dispositif d'actionnement.

Selon le mode dudit procédé, si les contraintes mécaniques subies par le dispositif d'actionnement sont supérieures à un seuil de contraintes déterminé, un organe de réduction des contraintes est actionné de sorte à diminuer lesdites contraintes dans le dispositif d'actionnement tout en maintenant la compression exercée par la manchette au-dessus du seuil de détection.

**BREVE DESCRIPTION DES DESSINS**

D'autres caractéristiques et avantages de l'invention ressortiront de la description détaillée qui va suivre, en référence aux dessins annexés sur lesquels :

- la figure 1 est une vue schématique de l'introduction d'une sonde urétrale chez un patient équipé d'un sphincter urinaire artificiel,
- la figure 2 illustre de manière schématique la mesure d'un paramètre de posture du patient,
- la figure 3 illustre l'architecture générale du système de détection selon un mode de réalisation de l'invention,

- les figures 4 à 6 illustrent schématiquement différentes configurations du système de détection et du sphincter urinaire artificiel,
- la figure 7 est un logigramme présentant les étapes du procédé de détection de l'introduction d'un dispositif endo-urétral chez un patient porteur d'un sphincter urinaire artificiel,
- la figure 8 illustre schématiquement une configuration d'un sphincter urinaire artificiel comprenant un organe de réduction des contraintes mécaniques subies par le dispositif d'actionnement.

**DESCRIPTION DETAILLEE DE MODES DE REALISATION DE L'INVENTION**

Sphincter urinaire artificiel

D'une manière générale, un sphincter artificiel comprend une manchette occlusive destinée à être implantée autour de l'urètre, ou éventuellement du col vésical chez la femme ou de la prostate chez l'homme, de sorte à comprimer l'urètre pour éviter des fuites urinaires, notamment dans le cas où le patient souffre d'incontinence d'effort.

Le sphincter artificiel comprend également un dispositif d'actionnement pour ajuster la compression exercée par la manchette.

Dans les sphincters artificiels les plus anciens, ce dispositif d'actionnement était commandé manuellement par le patient, par exemple par une pression exercée sur un dispositif de pompe agencé sous la peau.

A l'heure actuelle, des systèmes plus perfectionnés sont en cours de développement afin d'éviter au patient une pression manuelle sur la pompe pour contrôler la manchette.

Le sphincter artificiel comprend alors une unité de commande, également implantée dans le corps du patient, qui est adaptée pour commander le dispositif d'actionnement de la manchette.

Il existe actuellement différents sphincters artificiels, mettant en oeuvre différentes technologies de manchettes (hydraulique, mécanique, etc.) et différentes technologies d'actionnement associées (actionneur piézo-électrique, câbles, sangles, dispositifs à mémoire de forme, etc.).

Ces différents sphincters artificiels sont connus de l'homme du métier.

On pourra par exemple se référer aux documents suivants [1] à [4] cités plus haut ou encore aux documents [7] et [8].

L'invention n'est pas limitée à une technologie de sphincter particulière.

Dans la suite du texte, on considère à titre d'illustration le cas de l'introduction d'une sonde urétrale, mais l'invention s'applique à la détection de l'introduction de tout dispositif endo-urétral, quelle que soit la fonction de ce dispositif. Par « dispositif endo-urétral » on entend tout dispositif destiné à être inséré dans l'urètre d'un patient en vue d'accéder à l'urètre, au col vésical ou à la vessie, quelle que soit la finalité de ce dispositif. Parmi les dispositifs endo-urétraux on peut citer, outre les sondes, les dilatateurs, les fibroscopes qu'ils soient souples ou rigides.

La figure 1 illustre de manière schématique l'introduction d'une sonde urétrale chez un patient équipé d'un sphincter urinaire artificiel.

Le sphincter artificiel comprend une manchette occlusive 2 entourant la partie 1 à occlure (à savoir selon les cas l'urètre, le col vésical ou bien la prostate) du patient, et un boîtier de commande 7 également implanté dans le corps du patient (le repère 8 désignant la peau du patient).

Le boîtier est réalisé en un matériau biocompatible et est destiné à protéger les composants du sphincter autres que la manchette.

Ledit boîtier comprend le dispositif d'actionnement 5 de la manchette 1, qui est relié à la manchette par une liaison dont la nature dépend du mode d'actionnement de la manchette.

Par exemple, dans le cadre d'une manchette hydraulique, le dispositif d'actionnement comprend un réservoir et la liaison est une tubulure 17 remplie d'un fluide pouvant être transféré de manière bidirectionnelle de la manchette au réservoir selon que l'on souhaite augmenter ou diminuer la compression exercée. Dans le cadre d'une bande entourant l'urètre, le dispositif d'actionnement peut être un système électromécanique couplé à un câble 20 pour exercer une compression sur l'urètre.

Le boîtier 7 comprend en outre l'unité de commande 6, qui est reliée au dispositif d'actionnement par tout moyen approprié.

Lors d'un sondage urétral, on introduit un dispositif endo-urétral 3 dans l'urètre, incluant la partie entourée par la manchette.

Le système de détection décrit plus bas permet de relâcher (soit totalement, soit partiellement) la compression exercée par la manchette occlusive dès qu'est détectée l'introduction du dispositif endo-urétral.

Système de détection

A cet effet, le système de détection (qui n'est pas illustré sur la figure 1) comprend les composants suivants.

D'une part, le système comprend au moins un capteur dit « capteur de compression » adapté pour mesurer un paramètre de compression de l'urètre ou du col vésical.

Dans un souci de concision on parlera dans la suite du texte de compression de l'urètre, mais il va de soi que cela couvre également, le cas échéant, la compression du col vésical chez la femme ou de la prostate chez l'homme.

Ledit capteur peut mesurer la compression de l'urètre directement, c'est-à-dire en étant placé sur la paroi de l'urètre.

De manière alternative, ledit capteur peut mesurer la compression de l'urètre de manière indirecte, c'est-à-dire en mesurant une valeur de compression associée à l'un des composants du sphincter artificiel.

Ainsi, par exemple, dans le cas où le sphincter artificiel comprend une manchette hydraulique actionnée par un réservoir, le capteur de compression peut être un capteur de pression agencé contre une paroi dudit réservoir, de sorte à mesurer la pression dans le circuit hydraulique.

Une calibration permet d'établir la relation entre la pression du circuit hydraulique mesurée par le capteur et la compression exercée sur l'urètre.

On parle donc dans la suite du texte de « paramètre de compression de l'urètre » pour désigner le résultat de la mesure donné par le capteur, qui est représentatif de la compression de l'urètre.

L'introduction d'une sonde urétrale se traduit par la mesure, par ledit capteur, d'une valeur élevée de la compression exercée sur l'urètre.

On définit donc un seuil au-delà duquel la compression est considérée comme susceptible d'être due à l'introduction d'une sonde urétrale.

Ce seuil est déterminé de sorte à ce que ni la partie comprimée ni la manchette occlusive ne puissent être endommagées.

La compression n'est pas nécessairement mesurée en continu.

En effet, pour des raisons d'économie de l'énergie consommée par le capteur de compression, celui-ci peut être activé et désactivé périodiquement.

La fréquence de mesure de la compression résulte avantageusement d'un compromis entre une fréquence élevée assurant la réactivité du système de détection et une fréquence basse minimisant l'énergie consommée.

D'autre part, le système de détection comprend en outre au moins un capteur dit « capteur de posture » adapté pour mesurer un paramètre de posture du patient.

Par « posture », on entend l'activité du patient et/ou l'inclinaison du patient.

Parmi les capteurs de posture usuellement employés dans les systèmes implantables, on peut citer les accéléromètres, les gyroscopes et les inclinomètres, mais l'invention peut être mise en œuvre au moyen de tout autre capteur adéquat.

Selon un mode de réalisation, le capteur de posture est un accéléromètre à trois axes implanté dans le corps du patient.

Ainsi, les paramètres de posture du patient qu'un tel accéléromètre permet de fournir sont :

- d'une part, l'activité du patient, basée par exemple sur la norme accélérométrique |a|, donnée par la formule $\sqrt{a_x^2 + a_y^2 + a_z^2}$ où $a_x$, $a_y$ et $a_z$ sont les valeurs d'accélération mesurées pour chacun des axes x, y, z de l'accéléromètre (cf. figure 2),
- d'autre part, l'inclinaison du patient.

La détermination de l'inclinaison du patient peut être basée sur la mesure de la gravité par les trois axes de l'accéléromètre.

Par exemple, selon l'axe x, on détermine un angle $\alpha = \arcsin(a_{xpb})$ où $\alpha$ est l'angle mesuré et $a_{xpb}$ est l'accélération mesurée par l'accéléromètre et filtrée par un filtre passe-bas afin de récupérer uniquement la composante quasi-statique du signal, correspondant à la composante de la gravité.

A partir de la mesure de l'angle selon l'axe vertical 23 du patient et de la mesure de l'angle selon l'axe antéro-postérieur 22 du patient (cf. figure 2), il est possible d'identifier une posture représentative d'un sondage urétral, à savoir la posture décubitus dorsal, plus particulièrement, lorsque le patient a au moins le buste allongé sur le dos.

Il n'est pas nécessaire à cet égard de déterminer une inclinaison précise du corps du patient ; il suffit d'identifier si celui-ci est allongé, sur le dos ou sur le ventre.

De manière avantageuse, ledit capteur peut être installé dans le boîtier 7.

Ainsi, la prise en compte de la posture dans laquelle se trouve le patient alors qu'une valeur élevée de la compression est exercée sur l'urètre permet d'éviter une décompression intempestive de la manchette.

En effet, la seule prise en compte de cette valeur élevée ne permet pas de déterminer avec certitude qu'une sonde urétrale est introduite.

Il n'est pas à exclure, par exemple, que le patient soit dans une posture ou exerce une activité qui engendre une forte compression de l'urètre, sans qu'aucune sonde urétrale ne soit introduite.

Ainsi, lorsque le patient est assis et qu'il appuie fortement sur son périnée, la manchette occlusive, qui est située dans la région périnéale, subit une compression importante, qui se traduit par une pression importante dans le circuit hydraulique, et une forte compression de l'urètre peut être mesurée.

Le capteur de posture permet d'éviter de fausses détections de l'introduction d'une sonde urétrale.

En effet, on peut associer le sondage urétral à certaines postures spécifiques du patient, à savoir :

- le fait que le patient soit allongé,
- le cas échéant, fait que le patient soit sensiblement immobile.

Par conséquent, la combinaison de la détection :

- d'une compression élevée exercée sur l'urètre pendant une durée déterminée, suffisamment longue pour être significative, et
- d'une posture du patient correspondant à une situation de sondage urétral,

permet de déterminer avec un degré de certitude suffisamment grand qu'un sondage urétral est en train d'être pratiqué sur le patient.

Comme le capteur de compression, le capteur de posture n'est pas nécessairement activé en continu, afin de minimiser sa consommation énergétique.

Le système de détection comprend avantageusement une horloge permettant de mesurer la durée pendant laquelle une valeur de pression supérieure à un seuil déterminé est mesurée.

Par exemple, ledit seuil de durée peut être fixé à quelques secondes.

Le système de détection comprend par ailleurs une unité de traitement configurée pour recevoir, par une liaison filaire ou une liaison sans fil, les données de mesure des capteurs de compression et de posture et pour déterminer :

- à partir des données de mesure du paramètre de compression de l'urètre, si ledit paramètre dépasse un seuil prédéterminé pendant une durée prédéterminée, sans qu'un ordre de l'unité de commande soit envoyé au dispositif d'actionnement pour réduire la compression exercée par la manchette (condition (i)),
- à partir des données de mesure du paramètre de posture du patient, si le patient est dans une position allongée et sensiblement immobile (condition (ii)).

Si les conditions (i) et (ii) sont remplies, l'unité de traitement émet, à destination du dispositif d'actionnement, un ordre de réduction immédiate de la compression exercée par la manchette occlusive sur l'urètre.

Eventuellement, l'unité de traitement ou l'unité de commande est configurée pour émettre un signal sonore avant la réduction de la compression exercée par la manchette.

Ladite unité de traitement peut typiquement comprendre un microcontrôleur.

L'horloge mentionnée plus haut peut être incluse dans ledit microcontrôleur.

D'autre part, le microcontrôleur peut éventuellement comprendre d'autres horloges, notamment pour gérer l'activation et la désactivation des capteurs de compression et de posture, afin de limiter la consommation d'énergie de ces derniers.

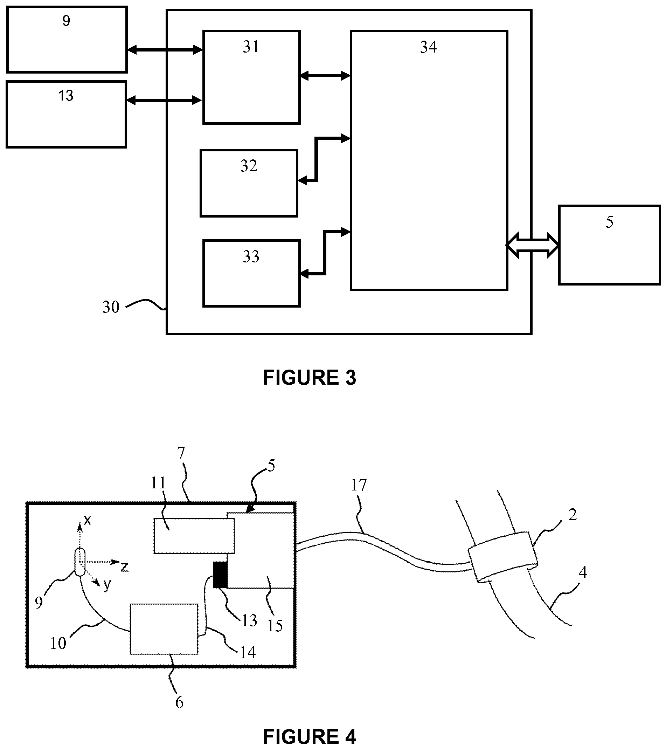

La figure 3 illustre une forme d'exécution de l'architecture générale du système de détection.

L'unité de traitement 30 communique avec le capteur de compression 13 et le capteur de posture 9 au moyen d'une interface 31.

La communication est schématisée par des flèches et peut être réalisée par une liaison filaire ou par une liaison sans fil, selon des protocoles connus.

L'unité de traitement 30 comprend en outre une mémoire 32 dans laquelle sont enregistrés les seuils et paramètres mentionnés plus haut.

L'unité de traitement comprend en outre une ou plusieurs horloges 33.

Un microcontrôleur 34 est relié à l'interface 31, la mémoire 32 et l'horloge 33.

Le microcontrôleur 34 est adapté pour implémenter un algorithme qui sera décrit en détail plus bas et pour communiquer avec le dispositif d'actionnement 5 de la manchette, de sorte à lui envoyer un ordre de décompression si les conditions d'un sondage urétral sont réunies.

Les figures 4 à 6 illustrent différents modes de réalisation non limitatifs du système de détection.

Dans ces trois modes de réalisation, l'unité de traitement est intégrée à l'unité de commande et n'est donc pas représentée en tant que telle.

Il va cependant de soi que l'unité de traitement pourrait être installée à un autre emplacement, dans le boîtier 7 ou à l'extérieur de celui-ci, sans pour autant sortir du cadre de la présente invention.

### Cas d'un sphincter comprenant un dispositif hydraulique d'actionnement de la manchette occlusive

La figure 4 illustre un exemple d'implémentation du système de détection pour un sphincter dans lequel la manchette 2 est une manchette hydraulique et le dispositif d'actionnement 5 de la manchette comprend un réservoir 15 à volume variable en liaison fluidique avec la manchette par une tubulure 17, ainsi qu'un mécanisme d'actionnement 11 permettant d'imposer un déplacement du fluide du réservoir 15 vers la manchette 2 ou inversement de la manchette 2 vers le réservoir 15.

Par exemple, le mécanisme d'actionnement 11 peut être un actionneur adapté pour faire varier le volume du réservoir 15 en générant un déplacement relatif de deux parois opposées.

Le capteur de compression 13 est un capteur de pression agencé contre une membrane (non représentée) agencée dans une paroi 15 du réservoir, de sorte à mesurer la pression dans le circuit hydraulique.

Il est relié par une liaison 14, filaire ou non, à l'unité de traitement intégrée dans l'unité de commande 6.

Le capteur de posture 9 est un accéléromètre à trois axes x, y, z, qui est relié par une liaison 10, filaire ou non, à l'unité de traitement intégrée dans l'unité de commande 6.

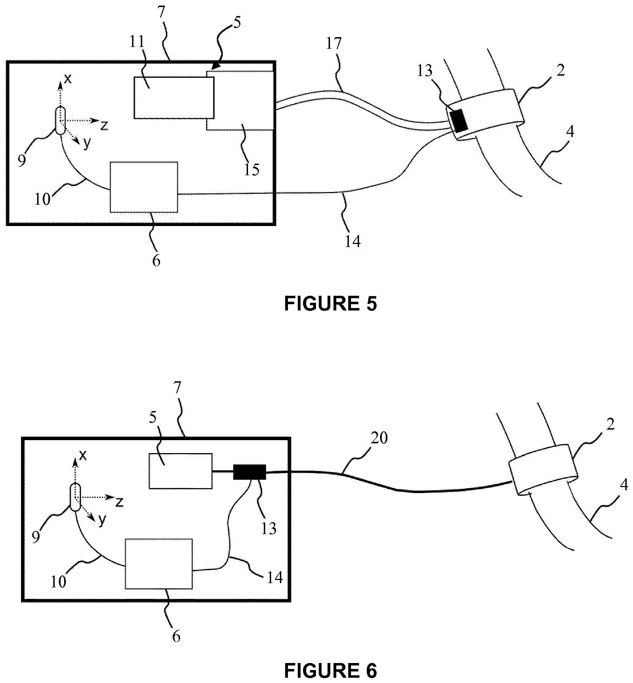

La figure 5 illustre une variante du mode de réalisation de la figure 4, dans lequel le capteur de pression est placé au contact de la manchette 2, de sorte à mesurer la pression dans ladite manchette.

<u>Cas d'un sphincter comprenant un dispositif d'actionnement par câble de la manchette occlusive</u>

La figure 6 illustre un exemple d'implémentation d'un système de détection pour un sphincter dans lequel, contrairement aux exemples décrits sur les figures 4 et 5, le dispositif d'actionnement de la manchette n'est pas basé sur un fonctionnement hydraulique mais mécanique.

Ledit dispositif est adapté pour ajuster une tension mécanique de la manchette occlusive.

La liaison entre la manchette 2 et le dispositif d'actionnement 5 peut ainsi être assurée par un câble 20.

Dans cette configuration, le capteur de compression 13 est un capteur capable de mesurer une force mécanique, par exemple la tension mécanique du câble 20.

Le capteur est ici représenté à l'intérieur du boîtier 7, mais il pourrait, de manière alternative, être placé à l'extérieur de celui-ci.

Comme dans l'exemple précédent, le capteur de posture 9 est un accéléromètre agencé dans le boîtier 7.

Les capteurs 9 et 13 sont reliés par des liaisons 10 et 14 à l'unité de traitement qui est intégrée à l'unité de commande 6.

<u>Procédé de détection</u>

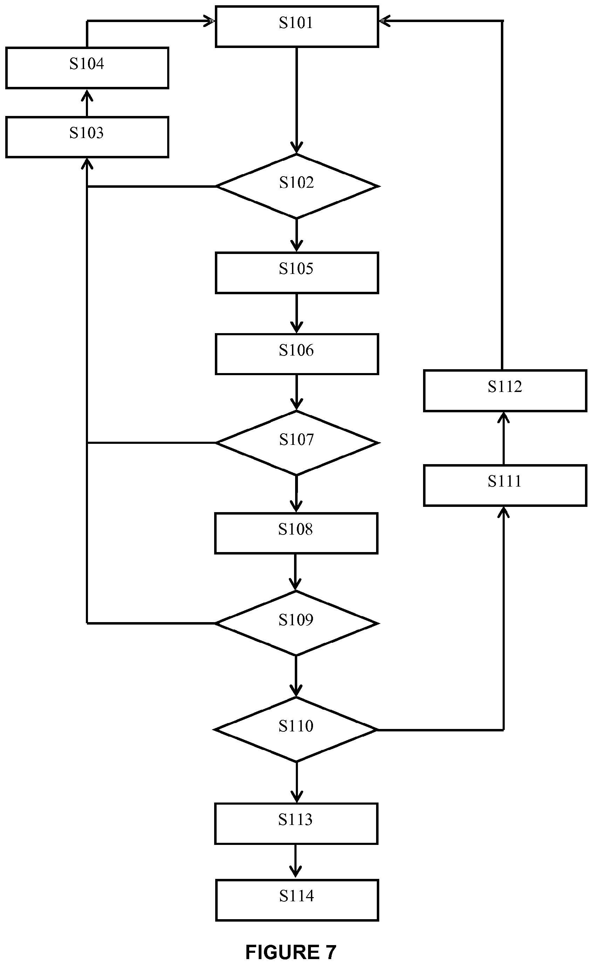

Les étapes du procédé de détection de l'introduction d'un dispositif endo-urétral chez un patient porteur du sphincter urinaire artificiel sont illustrées sur le logigramme de la figure 7. Ledit sphincter a été implanté chez le patient au préalable et l'invention ne couvre pas l'étape cette étape d'implantation préalable.

Cet algorithme est enregistré dans la mémoire du système de détection et implémenté par l'unité de traitement.

Naturellement, il ne s'agit que d'un exemple et l'on pourra ajouter des étapes, ou les implémenter de manière différente, sans sortir du cadre de l'invention.

Etape S101 : activation du capteur de compression ; lecture d'un signal d'un paramètre de compression de l'urètre provenant du capteur de compression ; désactivation du capteur une fois la mesure effectuée (cette séquence pouvant être répétée à plusieurs reprises pendant cette étape)

Etape S102 : comparaison du signal de compression avec un seuil prédéterminé,

Etape S105: si le paramètre de compression de l'urètre dépasse ledit seuil prédéterminé pendant une durée prédéterminée, activation du capteur de posture, lecture d'un signal d'un paramètre de posture du patient (typiquement, une ou plusieurs mesures d'accélération selon les trois axes de l'accéléromètre (composantes DC et AC) ; désactivation du capteur une fois la mesure effectuée ;

sinon retour à l'étape S101 en remettant à 0 un compteur (étape S103) et en attendant une durée prédéterminée (étape S104) avant de réaliser une nouvelle mesure ; cette attente peut durer de quelques millisecondes à quelques secondes,

Etape S106 : calcul de la norme accélérométrique à partir des mesures selon les trois axes de l'accéléromètre, en mettant en oeuvre un filtrage passe haut pour récupérer uniquement la composante AC du signal, traduisant une activité du patient,

Etape S107 : comparaison d'un paramètre dudit signal de posture (par exemple, la norme accélérométrique calculée précédemment) avec un plafond prédéterminé, et la détermination d'une position immobile du patient si ledit paramètre est inférieur audit plafond,

Etape S108 : mesure de l'angle d'inclinaison du patient selon l'axe vertical, afin de s'assurer qu'il est allongé (en effet, la mesure selon cet axe donne une accélération quasi-statique autour de 0 g, ce qui rend la mesure d'angle plus précise autour de cette valeur lorsque le patient est allongé) ; mesure de l'accélération quasi-statique selon l'axe antéro-postérieur du patient pour calculer l'angle d'inclinaison selon cet axe afin d'en déduire si le patient est sur le dos ou non,

Etape S109 : détermination, à partir dudit signal, de la position du patient en décubitus dorsal; à cet effet, on compare chaque angle d'inclinaison mesuré avec deux limites, chaque angle devant être compris entre ces deux limites,

Etape S110 : comparaison du compteur avec un nombre d'itérations prédéterminé ; si le compteur i est inférieur à ce nombre déterminé, répétition des étapes précédentes depuis l'étape S101 pendant un nombre prédéterminé d'itérations ; cet indice i permet de s'assurer pendant une durée prédéterminée que le patient est effectivement en train de subir un sondage urétral, afin d'éviter une fausse alarme et une décompression intempestive de la manchette ;

Etape S111 : attente d'une durée prédéterminée (cette attente, combinée au nombre d'itérations mesuré à l'étape S110 étant une condition de décision d'envoi d'un ordre de décompression à la manchette)

Etape S112 : incrémentation de 1 du nombre d'itérations,

Etape S113 : réinitialisation à 0 du compteur d'itérations,

Etape S114: émission, à destination du dispositif d'actionnement du sphincter urinaire artificiel, d'un ordre de réduction immédiate de la compression exercée par la manchette occlusive sur l'urètre.

La compression de la manchette occlusive après un sondage peut être réalisée soit automatiquement après un temps prédéterminé, soit manuellement par le patient ou le personnel soignant à partir par exemple d'une télécommande externe communiquant avec l'implant.

Une vérification par recompression progressive de la manchette et mesure de la pression de compression permet de s'assurer que la sonde est encore insérée ou a été retirée.

## Cas particulier d'un patient paraplégique

De manière avantageuse, la mesure du paramètre de posture du patient peut être mise à profit pour certains patients, notamment les patients paraplégiques, qui sont amenés à réaliser eux-mêmes des sondages urétraux en position assise.

Pour ces patients, il est donc nécessaire de prendre en compte le paramètre postural dans la détection du sondage urétral de différentes manières.

Comme expliqué plus haut, si le patient est couché et que la pression augmente dans la manchette, son ouverture complète sera automatiquement réalisée.

Si le patient est assis et que l'on détecte une augmentation de pression au niveau de la manchette, c'est que le patient est en train de se sonder volontairement.

Dans ce cas, la manchette peut être ouverte non pas complètement mais juste assez pour laisser passer la sonde et ainsi éviter des fuites d'urines entre l'urètre et la sonde.

Cet ajustement de la compression peut en effet prévenir l'apparition de fuites chez le patient assis pendant le sondage.

## Systèmes de sécurité

Dans le cas où la pression dans le circuit hydraulique ou le système mécanique devient très élevée et proche des limites définies dans les préconisations techniques concernant la tenue en pression des différents éléments du circuit hydraulique, respectivement du système mécanique, l'unité de traitement peut envoyer de manière automatique un ordre de décompression de la manchette, sans vérifier la posture du patient.

Dans ce cas, le seuil de sécurité est supérieur au seuil de détection par rapport auquel le paramètre de compression est évalué dans la condition (i).

Selon un mode de réalisation particulièrement avantageux de l'invention, le dispositif d'actionnement de la manchette comprend un organe de réduction des contraintes mécaniques causées par l'exercice d'une compression trop forte de l'urètre, permettant de protéger le dispositif d'actionnement des risques de détérioration pendant la période de détection de l'introduction d'une sonde urétrale.

Comme exposé précédemment, le procédé de détection comprend une période d'attente présentant une durée prédéterminée pendant laquelle la compression mesurée doit être supérieure au seuil de détection prédéfini pour que l'introduction d'une sonde soit identifiée. Cette période d'attente, relativement courte, permet d'éviter les fausses détections liées à des pics de compression intempestifs de l'urètre dans les conditions de détection.

Pendant cette courte période, les contraintes mécaniques subies par le dispositif d'actionnement résultant d'une compression importante de l'urètre peuvent devenir très importantes, ce qui pourrait causer la détérioration de l'une ou plusieurs des parties du dispositif d'actionnement. Dans le cas d'un système d'actionnement hydraulique, la pompe, la tubulure, la manchette, le capteur de pression et/ou les connecteurs peuvent être concernés par cette dégradation. Dans le cas d'un système d'actionnement mécanique, la manchette, le câble et/ou le capteur de tension mécanique du câble peuvent être affectés par ces contraintes trop importantes.

Afin d'éviter de détériorer le dispositif d'actionnement, l'organe de réduction des contraintes est conçu pour, lorsque les contraintes mécaniques (pression dans le circuit fluidique, tension mécanique du câble) dépassent un seuil de contraintes déterminé, absorber une partie desdites contraintes de sorte à réduire les contraintes subies par le dispositif d'actionnement.

L'organe de réduction des contraintes est dimensionné pour diminuer les contraintes jusqu'à un niveau dans lequel elles sont trop faibles pour risquer d'endommager le dispositif d'actionnement tout en étant suffisamment élevées pour ne pas relâcher totalement la compression exercée par la manchette. Plus précisément, dans la mesure où l'on détecte l'introduction d'une sonde urétrale par la comparaison de la compression avec un seuil prédéterminé, la compression exercée par la manchette après réduction des contraintes doit rester supérieure audit seuil de détection.

En fonction du mode de réduction des contraintes envisagé et de la structure du dispositif d'actionnement, l'homme du métier est en mesure de concevoir un organe remplissant ces conditions.

Selon un mode de réalisation, dans le cas d'un système hydraulique, l'organe de réduction des contraintes comprend une chambre d'expansion agencée dans le circuit hydraulique et se déclenchant mécaniquement lorsque la pression dans le circuit fluidique dépasse un seuil défini. Cela a pour effet de transférer une partie du fluide du circuit hydraulique vers la chambre d'expansion afin de réduire la pression dans ce dernier.

De manière alternative, l'organe de réduction des contraintes peut comprendre un piston couplé à un système de ressort ayant une raideur suffisamment élevée pour rester sensiblement fixe lorsque la pression dans le circuit hydraulique correspond à la pression de fonctionnement normal du sphincter artificiel (c'est-à-dire typiquement inférieure à 200 cm $H_2O$) et mobile sous l'effet d'une pression plus élevée.

L'organe de réduction des contraintes n'a pas pour but de réduire suffisamment la pression dans la manchette occlusive pour autoriser le passage d'une sonde dans l'urètre au niveau de la manchette, mais permet de protéger le circuit hydraulique lorsque de contraintes élevées sont appliqués sur ce dernier, notamment lors des premiers instants du passage d'un dispositif endo-urétral. Cet organe est réalisé de sorte à avoir un effet minime lors du fonctionnement normal du sphincter artificiel.

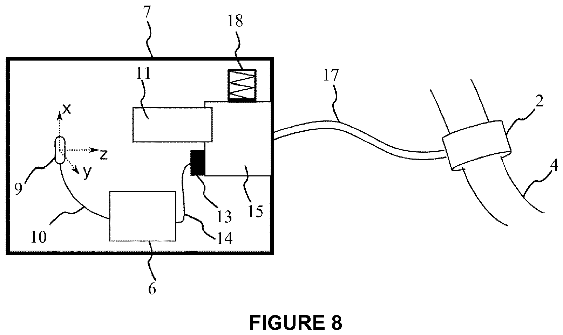

La figure 8 illustre un schéma de principe d'un système de détection pour un sphincter artificiel dans lequel le dispositif d'actionnement de la manchette est hydraulique. Les éléments remplissant la même fonction que ceux représentés sur les figures 4 et 5 sont désignés par les mêmes signes de référence et ne sont pas décrits à nouveau.

L'organe de réduction des contraintes est désigné par le repère 18. Sur cette figure, il est représenté agencé sur le réservoir 15 mais il pourrait être agencé en tout autre emplacement du circuit hydraulique ou de la manchette occlusive.

L'organe de réduction des contraintes peut présenter différentes formes d'exécution ; par exemple et de manière non limitative :

- un clapet couplé avec un ressort ou un matériau spécifique dans une chambre d'expansion ;
- un composant du circuit hydraulique réalisé en un matériau souple ayant la propriété de se déformer à partir d'un certain seuil de pression ;
- une membrane déformable au-delà d'un certain seuil de pression ou en fonction de la pression appliquée ;
- le réservoir 15 conçu de manière à être déformable au-delà d'un certain seuil de pression ou en fonction de la pression appliquée ;
- le mécanisme d'actionnement 11 conçu de manière à être déformable au-delà d'un certain seuil de pression ou en fonction de la pression appliquée.

A titre d'exemple, l'organe de réduction des contraintes peut fonctionner de la manière suivante en lien avec la détection de l'introduction d'une sonde urétrale :
Lorsqu'une sonde est introduite dans l'urètre, la pression dans le circuit hydraulique est telle que l'organe de réduction des contraintes se déclenche, conduisant à diminuer la pression dans le circuit hydraulique jusqu'à une valeur supérieure au seuil de détection mais inférieure à la contrainte maximale supportée par le dispositif d'actionnement.

On vérifie que la pression dans le circuit hydraulique est supérieure au seuil de détection pendant une durée déterminée.

A partir des données de mesure du paramètre de posture du patient, on détermine si le patient est dans une position allongée et, le cas échéant, immobile.

Si les conditions de pression et de posture sont remplies, on détecte l'introduction d'un dispositif endo-urétral.

Selon une autre forme d'exécution, l'organe de réduction des contraintes peut également être appliqué à un sphincter artificiel basé sur un système d'actionnement mécanique. Dans ce cas, ledit organe peut notamment comprendre un mécanisme placé sur le câble (par exemple, un système de ressort à raideur élevé) utilisé pour absorber les efforts trop importants lors des premiers instants du passage d'une sonde endo-urétrale, avant la détection et la décompression du dispositif occlusif.

Enfin, il va de soi que les exemples que l'on vient de donner ne sont que des illustrations particulières en aucun cas limitatives quant aux domaines d'application de l'invention.

## REFERENCES

[1] WO 2009/027196
[2] Development of a Novel Artificial Urinary Sphincter, H. Lamraoui et al, IEEE/ASME Transactions on Mechatronics, Vol. 15, No. 6, Dec. 2010
[3] US 6,162,238
[4] US 5,704,893
[5] The artificial urinary sphincter and urinary catheterization: What every physician should know and do to avoid serious complications, T.L. Mulholland et al, International Urology and Nephrology 36: 197-201, 2004
[6] Indications for Revision of Artificial Urinary Sphincter and Modifiable Risk Factors for Device-Related Morbidity, 1.1. Anusionwu et al, Neurourology and Urodynamics, Wiley, 2012
[7] US 5,509,888
[8] US 6,135,945

**Revendications**

1. Système de détection d'un dispositif endo-urétral pour un sphincter urinaire artificiel implantable dans le corps d'un patient, ledit sphincter comprenant une manchette occlusive (2) adaptée pour comprimer l'urètre, respectivement le col vésical ou la prostate dudit patient, un dispositif d'actionnement (5) de ladite manchette (2) pour ajuster la compression exercée par la manchette et une unité de commande (6) adaptée pour commander le dispositif d'actionnement,
ledit système de détection comprend :

   - au moins un capteur (13) dit « capteur de compression » adapté pour mesurer un paramètre de compression de l'urètre, respectivement du col vésical ou de la prostate,
   - au moins un capteur (9) dit « capteur de posture » adapté pour mesurer un

paramètre de posture du patient, ledit système de détection étant **caractérisé en ce qu'**il comprend :

- une unité de traitement (30) configurée pour :

- recevoir des données de mesure desdits capteurs (9, 13),
- déterminer, à partir des données de mesure du paramètre de compression de l'urètre, respectivement du col vésical :

(i) si le paramètre de compression de l'urètre, respectivement du col vésical, dépasse un seuil prédéterminé, dit « seuil de détection », pendant une durée prédéterminée, sans qu'un ordre de ladite unité de commande soit envoyé au dispositif d'actionnement (5) pour réduire la compression exercée par la manchette (2),

- déterminer, à partir des données de mesure du paramètre de posture du patient :
(ii) si le patient est dans une position allongée,
- si les conditions (i) et (ii) sont remplies, émettre, à destination du dispositif d'actionnement (5) du sphincter urinaire artificiel, un ordre de réduction immédiate de la compression exercée par la manchette (2) occlusive sur l'urètre, respectivement sur le col vésical ou la prostate,

- un organe (18) de réduction des contraintes mécaniques subies par le dispositif d'actionnement conçu pour, lorsque les contraintes mécaniques subies par le dispositif d'actionnement dépassent un seuil de contraintes déterminé, absorber une partie desdites contraintes de sorte à réduire les contraintes subies par le dispositif d'actionnement tout en maintenant la compression exercée par la manchette au-dessus du seuil de détection.

2. Système selon la revendication 1, **caractérisé en ce que** ledit au moins un capteur de posture et l'unité de traitement sont configurés pour déterminer si le patient est dans la position décubitus dorsal et pour déterminer si ledit patient est sensiblement immobile.

3. Système selon l'une des revendications 1 ou 2, **caractérisé en ce que** ledit au moins un capteur de posture est choisi parmi un accéléromètre, un gyroscope et un inclinomètre.

4. Système selon l'une des revendications 1 à 3, **caractérisé en ce que** le capteur de compression est adapté pour mesurer un paramètre de fonctionnement du dispositif d'actionnement de la manchette et **en ce que** l'unité de traitement est configurée pour déterminer, à partir de la mesure dudit paramètre, la compression de l'urètre.

5. Système selon l'une des revendications 1 à 4, **caractérisé en ce que** le dispositif d'actionnement de la manchette est un dispositif hydraulique adapté pour ajuster une pression de fluide dans la manchette occlusive.

6. Système selon la revendication 5, **caractérisé en ce que** ledit au moins un capteur de compression est un capteur de pression agencé pour mesurer la pression de fluide dans ledit dispositif hydraulique.

7. Système selon l'une des revendications 1 à 4, **caractérisé en ce que** le dispositif d'actionnement de la manchette est un dispositif adapté pour ajuster une tension mécanique de la manchette occlusive.

8. Système selon la revendication 7, **caractérisé en ce que** le capteur de compression est un capteur de contrainte mécanique.

9. Système selon la revendication 4, dans lequel ledit organe (18) de réduction des contraintes comprend une chambre d'expansion, ladite chambre d'expansion étant agencée dans le dispositif hydraulique de sorte à transférer une partie du volume de fluide dans ladite chambre d'expansion de manière à réduire à une valeur définie la pression dans le dispositif hydraulique.

10. Système selon l'une des revendications 1 à 9, **caractérisé en ce que** l'unité de traitement ou l'unité de commande est configurée pour émettre un signal sonore avant la réduction de la compression exercée par la manchette.

11. Système selon l'une des revendications 1 à 10, **caractérisé en ce que** l'unité de traitement comprend une mémoire sur laquelle est enregistré un programme d'ordinateur, ledit programme comprenant des instructions pour la mise en oeuvre des étapes suivantes :

(S101) la lecture d'un signal d'un paramètre de compression de l'urètre, respectivement du col vésical,
(S102) la comparaison dudit signal avec le seuil de détection prédéterminé,
(S105) si le paramètre de compression de l'urètre, respectivement du col vésical, dépasse ledit seuil prédéterminé pendant une durée prédéterminée, la lecture d'un signal d'un paramètre de posture du patient, sinon retour à l'étape S101,

(S107) la comparaison d'un paramètre dudit signal avec un plafond prédéterminé, et la détermination d'une position immobile du patient si ledit paramètre est inférieur audit plafond,

(S109) la détermination, à partir dudit signal, de la position du patient en décubitus dorsal,

(S110) la répétition des étapes précédentes pendant un nombre prédéterminé d'itérations,

(S114) l'émission, à destination du dispositif d'actionnement du sphincter urinaire artificiel, d'un ordre de réduction immédiate de la compression exercée par la manchette occlusive sur l'urètre, respectivement sur le col vésical ou la prostate.

**12.** Système selon l'une des revendications 1 à 11, **caractérisé en ce que** ledit au moins un capteur de posture et l'unité de traitement sont configurés pour déterminer si le patient est dans une position assise et pour déterminer si le buste dudit patient est sensiblement immobile, et **en ce que** la condition (ii) pour l'émission de l'ordre de réduction immédiate de la compression exercée par la manchette (2) est lorsque le patient est assis.

**13.** Système selon l'une des revendications 1 à 12, **caractérisé en ce que** l'unité de traitement est configurée pour, si ledit paramètre de compression de l'urètre dépasse un seuil de sécurité prédéterminé supérieur au seuil de détection par rapport auquel ledit paramètre est comparé dans l'évaluation de la condition (i), émettre un ordre de réduction immédiate de la compression exercée par la manchette (2), quel que soit le statut de la condition (ii).

**14.** Sphincter urinaire artificiel comprenant :

- une manchette occlusive (2) adaptée pour comprimer l'urètre, respectivement le col vésical ou la prostate d'un patient dans lequel il est destiné à être implanté,
- un dispositif d'actionnement (5) de ladite manchette pour ajuster la compression exercée par la manchette,
- une unité de commande (6) adaptée pour commander le dispositif d'actionnement, et
- un système de détection selon l'une des revendications 1 à 13 communiquant avec le dispositif d'actionnement (5).

**Patentansprüche**

**1.** System zur Erkennung einer endo-urethralen Vorrichtung für einen artifiziellen Harnsphinkter, der in den Körper eines Patienten implantierbar ist, wobei der Sphinkter eine okklusive Manschette (2), die dazu geeignet ist, die Harnröhre beziehungsweise den Blasenhals oder die Prostata des Patienten zu komprimieren, eine Vorrichtung (5) zur Betätigung der Manschette (2) zum Anpassen der von der Manschette ausgeübten Kompression und eine Steuerungseinheit (6) umfasst, die dazu geeignet ist, die Betätigungsvorrichtung zu steuern, wobei das Erkennungssystem umfasst:

- mindestens einen "Kompressionssensor" genannten Sensor (13), der dazu geeignet ist, einen Kompressionsparameter der Harnröhre, beziehungsweise des Blasenhalses oder der Prostata zu messen,
- mindestens einen "Haltungssensor" genannten Sensor (9), der dazu geeignet ist, einen Haltungsparameter des Patienten zu messen, wobei das Erkennungssystem **dadurch gekennzeichnet ist, dass** es umfasst:
- eine Verarbeitungseinheit (30), die ausgestaltet ist zum:

- Empfangen von Messdaten der Sensoren (9, 13),
- ausgehend von den Messdaten des Kompressionsparameters der Harnröhre beziehungsweise des Blasenhalses, Bestimmen:

(i) ob der Kompressionsparameter der Harnröhre beziehungsweise des Blasenhalses während einer vorbestimmten Dauer einen vorbestimmten, "Erkennungsschwellenwert" genannten Schwellenwert überschreitet, ohne dass ein Befehl zum Vermindern der von der Manschette (2) ausgeübten Kompression von der Steuerungseinheit an die Betätigungsvorrichtung (5) gesendet wurde,

- ausgehend von den Messdaten des Haltungsparameters des Patienten, Bestimmen:
(ii) ob der Patient sich in einer liegenden Position befindet,
- wenn die Bedingungen (i) und (ii) erfüllt sind, Senden eines Befehls zur sofortigen Verminderung der von der okklusiven Manschette (2) auf die Harnröhre beziehungsweise den Blasenhals oder die Prostata ausgeübten Kompression an die Vorrichtung (5) zur Betätigung des artifiziellen Harnsphinkters,

- ein Organ (18) zur Verminderung der mechanischen Beanspruchungen, denen die Betätigungsvorrichtung unterliegt, das gestaltet ist, um, wenn die mechanischen Beanspruchungen, denen die Betätigungsvorrichtung unterliegt, einen bestimmten Schwellenwert für Beanspruchungen überschreiten, einen Teil der Beanspruchungen aufzunehmen, derart dass die Beanspruchungen, denen die Betätigungsvorrichtung unterliegt, vermindert werden und dabei die Kompression, die von der Manschette ausgeübt wird, oberhalb des Erkennungsschwellenwerts gehalten wird.

2. System nach Anspruch 1, **dadurch gekennzeichnet, dass** der mindestens eine Haltungssensor und die Verarbeitungseinheit ausgestaltet sind, um zu bestimmen, ob der Patient sich in der Rückenlage befindet, und um zu bestimmen, ob der Patient im Wesentlichen bewegungslos ist.

3. System nach einem der Ansprüche 1 oder 2, **dadurch gekennzeichnet, dass** der mindestens eine Haltungssensor aus einem Beschleunigungsmesser, einem Gyroskop und einem Neigungsmesser ausgewählt ist.

4. System nach einem der Ansprüche 1 bis 3, **dadurch gekennzeichnet, dass** der Kompressionssensor dazu geeignet ist, einen Betriebsparameter der Vorrichtung zur Betätigung der Manschette zu messen, und dadurch, dass die Verarbeitungseinheit ausgestaltet ist, um ausgehend von der Messung des Parameters die Kompression der Harnröhre zu bestimmen.

5. System nach einem der Ansprüche 1 bis 4, **dadurch gekennzeichnet, dass** die Vorrichtung zur Betätigung der Manschette eine hydraulische Vorrichtung ist, die dazu geeignet ist, einen Fluiddruck in der okklusiven Manschette anzupassen.

6. System nach Anspruch 5, **dadurch gekennzeichnet, dass** der mindestens eine Kompressionssensor ein Drucksensor ist, der eingerichtet ist, um den Fluiddruck in der hydraulischen Vorrichtung zu messen.

7. System nach einem der Ansprüche 1 bis 4, **dadurch gekennzeichnet, dass** die Vorrichtung zur Betätigung der Manschette eine Vorrichtung ist, die dazu geeignet ist, eine mechanische Spannung der okklusiven Manschette anzupassen.

8. System nach Anspruch 7, **dadurch gekennzeichnet, dass** der Kompressionssensor ein Sensor für die mechanische Beanspruchung ist.

9. System nach Anspruch 4, wobei das Organ (18) zur Verminderung der Beanspruchungen eine Expansionskammer umfasst, wobei die Expansionskammer derart in der hydraulischen Vorrichtung eingerichtet ist, dass ein Teil des Fluidvolumens in der Expansionskammer derart übertragen wird, dass der Druck in der hydraulischen Vorrichtung auf einen definierten Wert vermindert wird.

10. System nach einem der Ansprüche 1 bis 9, **dadurch gekennzeichnet, dass** die Verarbeitungseinheit oder die Steuerungseinheit ausgestaltet ist, um vor der Verminderung der von der Manschette ausgeübten Kompression ein akustisches Signal auszugeben.

11. System nach einem der Ansprüche 1 bis 10, **dadurch gekennzeichnet, dass** die Verarbeitungseinheit einen Speicher umfasst, in dem ein Computerprogramm aufgezeichnet ist, wobei das Programm Anweisungen für das Durchführen der folgenden Schritte umfasst:

   (S101) Lesen eines Signals eines Kompressionsparameters der Harnröhre beziehungsweise des Blasenhalses,
   (S102) Vergleichen des Signals mit dem vorbestimmten Erkennungsschwellenwert,
   (S105) wenn der Kompressionsparameter der Harnröhre beziehungsweise des Blasenhalses den vorbestimmten Schwellenwert während einer vorbestimmten Dauer überschreitet, Lesen eines Signals eines Haltungsparameters des Patienten, wenn nicht, Zurückkehren zum Schritt S101,
   (S107) Vergleichen eines Parameters des Signals mit einer vorbestimmten Grenze und Bestimmen einer unbewegten Position des Patienten, wenn der Parameter niedriger als die Grenze ist,
   (S109) ausgehend von dem Signal, Bestimmen, dass der Patient sich in der Rückenlage befindet,
   (S110) Wiederholen der vorhergehenden Schritte während einer vorbestimmten Anzahl von Iterationen,
   (S114) Ausgeben eines Befehls zur sofortigen Verminderung der von der okklusiven Manschette auf die Harnröhre, beziehungsweise auf den Blasenhals oder die Prostata ausgeübten Kompression an die Vorrichtung zur Betätigung des artifiziellen Harnsphinkters.

12. System nach einem der Ansprüche 1 bis 11, **dadurch gekennzeichnet, dass** der mindestens eine Haltungssensor und die Verarbeitungseinheit ausgestaltet sind, um zu bestimmen, ob der Patient sich in einer sitzenden Position befindet, und um zu bestimmen, ob der Oberkörper des Patienten im Wesentlichen unbewegt ist, und dadurch, dass die Bedingung (ii) für die Ausgabe des Befehls zur sofortigen Verminderung der von der Manschette (2) ausgeübten Kompression ist, wenn der Patient sitzt.

**13.** System nach einem der Ansprüche 1 bis 12, **dadurch gekennzeichnet, dass** die Verarbeitungseinheit ausgestaltet ist, um, wenn der Kompressionsparameter der Harnröhre einen vorbestimmten Sicherheitsschwellenwert überschreitet, der höher ist als der Erkennungsschwellenwert, mit dem der Parameter bei der Bewertung der Bedingung (i) verglichen wird, unabhängig vom Zustand der Bedingung (ii) einen Befehl zur sofortigen Verminderung der von der Manschette (2) ausgeübten Kompression auszugeben.

**14.** Artifizieller Harnsphinkter, der umfasst:

- eine okklusive Manschette (2), die dazu geeignet ist, die Harnröhre beziehungsweise den Blasenhals oder die Prostata eines Patienten zu komprimieren, in der/dem er bestimmt ist, platziert zu werden,
- eine Vorrichtung zur Betätigung (5) der Manschette zum Anpassen der von der Manschette ausgeübten Kompression,
- eine Steuerungseinheit (6), die dazu geeignet ist, die Betätigungsvorrichtung zu steuern, und
- ein Detektionssystem nach einem der Ansprüche 1 bis 13, das mit der Betätigungsvorrichtung (5) kommuniziert.

**Claims**

**1.** A system for detecting an endo-urethral device for an artificial urinary sphincter implantable in the body of a patient, said sphincter comprising an occlusive cuff (2) adapted to compress the urethra, respectively the bladder neck or the prostate of said patient, an activation device (5) of said cuff (2) for adjusting the compression exerted by the cuff and a control unit (6) adapted to control the activation device, said detection system comprises:

- at least one sensor (13) called « compression sensor » adapted to measure a compression parameter of the urethra, respectively of the bladder neck or of the prostate,
- at least one sensor (9) called « posture sensor » adapted to measure a posture parameter of the patient,

said detection system being **characterized in that** it comprises:

- a processing unit (30) configured to:

- receive measurement data from said sensors (9, 13),
- determine from the measurement data of the compression parameter of the urethra, respectively of the bladder neck:

(i) if the compression parameter of the urethra, respectively of the bladder neck, exceeds a predetermined threshold, called « detection threshold », during a predetermined period, without an order of said control unit being sent to the activation device (5) to reduce the compression exerted by the cuff (2),

- determine from the measurement data of the posture parameter of the patient:
(ii) if the patient is in a recumbent position,
- if the conditions (i) and (ii) are fulfilled, to send to the activation device (5) of the artificial urinary sphincter, an immediate reduction order of the compression exerted by the occlusive cuff (2) on the urethra, respectively on the bladder neck or the prostate,

- an element (18) for reducing mechanical stresses undergone by the activation device designed so as, when mechanical stresses undergone by the activation device exceed a determined stress threshold, to absorb some of said stresses so as to reduce stresses undergone by the activation device while maintaining the compression exerted by the cuff above the detection threshold.

**2.** The system according to claim 1, **characterized in that** said at least one posture sensor and the processing unit are configured to determine if the patient is in the decubitus dorsal position and to determine if said patient is substantially immobile.

**3.** The system according to one of claims 1 or 2, **characterized in that** said at least one posture sensor is selected from an accelerometer, a gyroscope and an inclinometer.

**4.** The system according to one of claims 1 to 3, **characterized in that** the compression sensor is adapted to measure an operating parameter of the activation device of the cuff and **in that** the processing unit is configured to determine, from the measurement of said parameter, the compression of the urethra.

**5.** The system according to one of claims 1 to 4, **characterized in that** the activation device of the cuff is a hydraulic device adapted for adjusting pressure of fluid in the occlusive cuff.

**6.** The system according to claim 5, **characterized in that** said at least one compression sensor is a pressure sensor arranged to measure the pressure of fluid in said hydraulic device.

**7.** The system according to one of claims 1 to 4, **characterized in that** the activation device of the cuff is a device adapted for adjusting mechanical tension of the occlusive cuff.

**8.** The system according to claim 7, **characterized in that** the compression sensor is a mechanical stress sensor.

**9.** The system according to claim 4, wherein said stress-reduction element (18) comprises an expansion chamber, said expansion chamber being arranged in the hydraulic device so as to transfer some of the volume of fluid in said expansion chamber to reduce pressure in the hydraulic device to a defined value.

**10.** The system according to one of claims 1 to 9, **characterized in that** the processing unit or the control unit is configured to send an audio signal prior to reduction of the compression exerted by the cuff.

**11.** The system according to one of claims 1 to 10, **characterized in that** the processing unit comprises a memory on which a computer program is recorded, said program comprising instructions for executing the following steps:

(S101) reading of a signal of a compression parameter of the urethra, respectively of the bladder neck,
(S102) comparison of said signal with the predetermined detection threshold,
(S105) if the compression parameter of the urethra, respectively of the bladder neck, exceeds said predetermined threshold during a predetermined period, reading of a signal of a posture parameter of the patient, if not return to step S101,
(S107) comparison of a parameter of said signal with a predetermined upper limit, and determination of an immobile position of the patient if said parameter is under said upper limit,
(S109) determination, from said signal, of the position of the patient in decubitus dorsal,
(S110) repetition of the preceding steps during a predetermined number of iterations,
(S114) sending, to the activation device of the artificial urinary sphincter, of an immediate reduction order of the compression exerted by the occlusive cuff on the urethra, respectively on the bladder neck or the prostate.

**12.** The system according to one of claims 1 to 11, **characterized in that** said at least one posture sensor and the processing unit are configured to determine if the patient is in a seated position and to determine if the torso of said patient is substantially immobile, and **in that** the condition (ii) for sending of the immediate reduction order of the compression exerted by the cuff (2) is when the patient is seated.

**13.** The system according to one of claims 1 to 12, **characterized in that** the processing unit is configured, if said compression parameter of the urethra exceeds a predetermined safety threshold greater than the detection threshold relative to which said parameter is compared in the evaluation of the condition (i), to send an immediate reduction order of the compression exerted by the cuff (2), irrespective of the status of the condition (ii).

**14.** An artificial urinary sphincter comprising:

- an occlusive cuff (2) adapted to compress the urethra, respectively the bladder neck or the prostate of a patient in whom it is intended to be implanted,
- an activation device (5) of said cuff for adjusting the compression exerted by the cuff,
- a control unit (6) adapted to control the activation device, and
- a detection system according to one of claims 1 to 13 communicating with the activation device (5).

**FIGURE 1**

**FIGURE 2**

**FIGURE 3**

**FIGURE 4**

**FIGURE 5**

**FIGURE 6**

**FIGURE 7**

**FIGURE 8**

## RÉFÉRENCES CITÉES DANS LA DESCRIPTION

**Documents brevets cités dans la description**

- WO 0150833 A
- WO 2009027196 A
- US 6162238 A
- US 5704893 A
- US 5509888 A
- US 6135945 A

**Littérature non-brevet citée dans la description**

- **H. LAMRAOUI et al.** Development of a Novel Artificial Urinary Sphincter. *IEEE/ASME Transactions on Mechatronics,* Décembre 2010, vol. 15 (6
- **T.L. MULHOLLAND et al.** The artificial urinary sphincter and urinary catheterization: What every physician should know and do to avoid serious complications. *International Urology and Nephrology,* 2004, vol. 36, 197-201
- Indications for Revision of Artificial Urinary Sphincter and Modifiable Risk Factors for Device-Related Morbidity, 1.1. **ANUSIONWU et al.** Neurourology and Urodynamics. Wiley, 2012